# FASCICULE DE BREVET EUROPEEN

(11) **EP 0 863 897 B1**
(45) Date de publication et mention de la délivrance du brevet: **16.06.1999**
(21) Numéro de dépôt: 96937390.1
(22) Date de dépôt: 05.11.1996
(51) Int. Cl.: C07D 413/04, C07D 413/14, A61K 31/445

(54) **DERIVES DE 5-PHENYL-3-(PIPERIDIN-4-YL)-1,3,4-OXADIAZOL-2(3H)-ONE, UTILES COMME LIGANDS DES RECEPTEURS 5-HT4 OU H3**
5-(PHENYL)-3-(4-PIPERIDINYL)-1,3,4-OXADIAZOL-2(3H)-ON-DERIVATE VERWENDBAR ALS 5-HT4- ODER H3-REZEPTORLIGANDEN
5-PHENYL-3-(PIPERIDIN-4-YL)-1,3,4-OXADIAZOL-2(3H)-ONE DERIVATIVES FOR USE AS 5-HT4- OR H3-RECEPTOR LIGANDS

(30) Priorité: 09.11.1995 FR 9513252; 09.11.1995 FR 9513253; 04.03.1996 FR 9602663
(43) Date de publication de la demande: 16.09.1998
(73) Titulaire: SYNTHELABO, 92350 Le Plessis Robinson (FR)
(72) Inventeur: JEGHAM, Samir, F-95100 Argenteuil (FR); LOCHEAD, Alistair, F-94220 Charenton (FR); GALLI, Frédéric, F-92420 Vaucresson (FR); NEDELEC, Alain, F-92700 Colombes (FR); SOLIGNAC, Axelle, F-75004 Paris (FR); DE CRUZ, Laurence, F-94340 Joinville-le-Pont (FR)
(74) Mandataire: Ludwig, Jacques
(86) Numéro de dépôt international: FR9601730
(87) Numéro de publication internationale: WO9717345

(56) Documents cités:
- WO-A-93/03725
- WO-A-93/16072
- WO-A-94/05654
- JP-A- 6 157 518

## Description

La présente invention a pour objet des composés répondant à la formule générale (I) 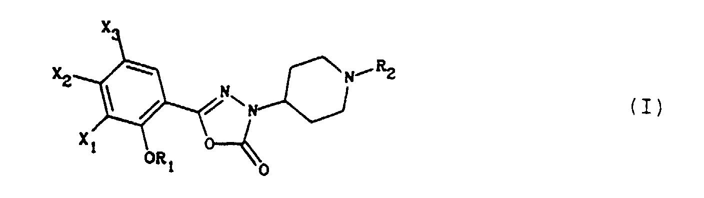 dans laquelle
R₁ représente un groupe (C₁-C₄)alkyle ou (C₃-C₇)cycloalkylméthyle,
X₁ représente un atome d'hydrogène ou d'halogène ou un groupe (C₁-C₄)alcoxy ou bien
OR₁ et X₁ représentent ensemble un groupe de formule -OCH₂O-, -O(CH₂)₂-, -O(CH₂)₃-, -O(CH₂)₂O- ou -O(CH₂)₃O-,
X₂ représente un atome d'hydrogène ou un groupe amino,
X₃ représente un atome d'hydrogène ou d'haiogène, et
R₂ représente soit un atome d'hydrogène, soit un groupe (C₁-C₆)alkyle éventuellement substitué, soit un groupe phényl(C₁-C₄)alkyle éventuellement substitué sur le noyau phényle, soit un groupe phényl(C₂-C₃)alcényle, soit un groupe phénoxy(C₂-C₄)alkyle, soit un groupe cyclo(C₃-C₇)alkylméthyle, soit un groupe 2,3-dihydro-1*H*-indén-1-yle ou 2,3-dihydro-1*H*-indén-2-yle, soit un groupe de formule générale -(CH₂)ₙCO-Z dans laquelle n représente un nombre de 1 à 6 et Z représente un groupe pipéridin-1-yle ou 4-(diméthylamino)pipéridin-1-yle.

Lorsque R₂ représente un groupe alkyle éventuellement substitué, un tel groupe est, de préférence, un groupe 2-éthoxy-2-oxoéthyle, un groupe 2-(diméthylamino)-2-oxoéthyle, un groupe 2-[(méthylsulfonyl)amino] éthyle, un groupe 2-oxo-2-phényléthyle, un groupe 2-hydroxy-2-phényiéthyle, un groupe butyle, un groupe 4,4,4-trifluorobutyle ou un groupe 4-trifluoro-3-hydroxybutyle.

Lorsque R₂ représente un groupe phényl(C₁-C₃)alkyle éventuellement substitué sur le noyau phényle, un tel groupe est, de préférence un groupe éventuellement substitué sur le noyau phényle par un atome d'halogène, par un groupe trifluorométhyle ou par un ou deux groupes méthoxy.

Lorsque R₂ représente un groupe de formule générale -(CH₂)ₙCO-Z, un tel groupe est, de préférence un groupe 4-oxo-4-(pipéridin-l-yl)butyle, un groupe 2-[4-(diméthylamino)pipéridin-1-yl] -2-oxoéthyle, un groupe 4-[4-(diméthylamino)pipéridin-1-yl]-4-oxobutyle, un groupe 5-[4-(diméthylamino)pipéridin-1-yl]-5-oxopentyle ou un groupe 6-[4-(diméthylamino)pipéridin-1-yl]-6-oxohexyle.

Les composés de l'invention peuvent exister à l'état de bases libres ou de sels d'addition à des acides. Par ailleurs, certains substituents R₂ contiennent un atome de carbone asymétrique ; les composés peuvent donc exister sous forme d'énantiomères purs ou de mélanges d'énantiomères.

Des composés de structure analogue à celle des composés de l'invention, et ayant probablement des effets thérapeutiques similaires, mais dont l'hétérocycle central est un 1,2,4-oxadiazole au lieu d'une 1,3,4-oxadiazol-2(3*H*)-one, sont décrits dans la demande de brevet JP-06 157 518, résumée dans *C. A.* **121**(23) 280649k.

Conformément à l'invention on peut préparer les composés de formule générale (I) par un procédé illustré par le schéma qui suit.

On fait réagir un ester de formule générale (II), dans laquelle R₁, X₁, X₂ et X₃ sont tels que définis ci-dessus et R₃ représente un groupe méthyle ou éthyle, avec l'hydrate d'hydrazine, en l'absence de solvant ou dans un solvant polaire protique, par exemple l'éthanol, pour obtenir un hydrazide de formule générale (III) dont on obtient la cyclisation en oxadiazole de formule générale (IV) soit au moyen de phosgène, dans un solvant aprotique, par exemple le dioxane, soit au moyen de chloroformiate de phényle, dans un solvant aprotique, par exemple le toluène. Lorsque, dans la formule générale (III), X₂ représente un groupe amino, ce dernier réagit avec le phosgène, et on estérifie le produit obtenu avec de l'alcool benzylique, le groupe amino étant ainsi protégé par un groupe benzyloxycarbonyle.
On fait ensuite réagir l'oxadiazole de formule générale (IV) 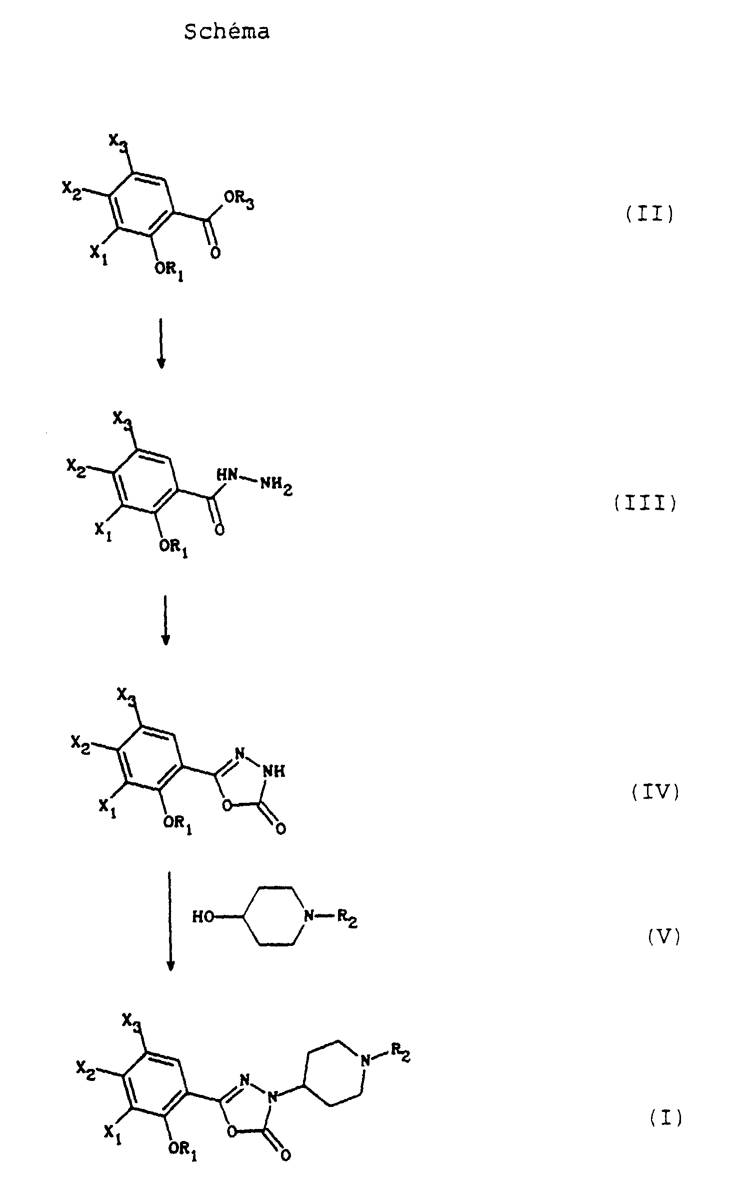 avec un pipéridin-4-ol de formule générale (V), dans laquelle R₂ est tel que défini à propos de la formule générale (I), mais différent d'un atome d'hydrogène, ou bien représente un groupe protecteur (1,1-diméthyléthoxy)carbonyle, en présence de triphénylphosphine et d'azodicarboxylate d'éthyle, dans un solvant aprotique, par exemple le tétrahydrofurane, puis s'il y a lieu, on déprotège l'azote du cycle pipéridine au moyen d'acide trifluoroacétique et, lorsque R₂ représente un atome d'hydrogène, et si on le désire, on fait réagir le composé obtenu avec un dérivé de formule générale R₂-X, dans laquelle X représente un groupe partant ou fonctionalisable, par exemple un atome d'halogène, un groupe méthanesulfonate, 4-méthylbenzènesulfonate ou une fonction carbonyle et R₂ est tel que défini à propos de la formule générale (I), mais différent d'un atome d'hydrogène, en présence de triéthylamine, dans un solvant aprotique, par exemple l'acétonitrile. Dans le cas particulier où R₂ représente un groupe 2,3-dihydro-1*H*-indényle, on effectue une amination réductive avec un composé de formule générale (I) dans laquelle R₂ représente un atome d'hydrogène et l'indanone correspondante.

Les esters de départ de formule générale (II), ou les acides correspondants, sont connus et décrits, notamment, dans les demandes de brevets EP-0231139, EP-0234872, WO-8403281, WO-9316072 et WO-9419344.

Les pipéridin-4-ols de formule générale (V) sont connus et/ou peuvent être préparés selon des méthodes analogues à celles décrites dans *J*. *Mol. Pharmacol*. (1992) **41**(4) 718-726 et dans les demandes de brevets WO-9303725 et EP-0309043.

Les exemples suivants illustrent en détail la préparation de quelques composés selon l'invention. Les microanalyses élémentaires et les spectres IR et RMN confirment les structures des composés obtenus. Les numéros des composés indiqués entre parenthèses dans les titres correspondent à ceux du tableau donné plus loin. Dans les noms des composés, le tiret "-" fait partie du mot, et le tiret "_" ne sert que pour la coupure en fin de ligne ; il est à supprimer en l'absence de coupure, et ne doit pas être remplacé par un tiret normal ou par un espace.

### Exemple 1 (Composé N°1).

### Bromhydrate de 5-(4-Amino-5-chloro-2-méthoxyphényl)-3-pipéridin-4-yl-1,3,4-oxadiazol-2(3H)-one.

### 1.1. Hydrazide de l'acide 4-amino-5-chloro-2-méthoxybenzoïque.

Dans un réacteur de 1 l on introduit 51,5 g (0,239 mole) de 4-amino-5-chloro-2-méthoxybenzoate de méthyle en suspension dans 460 ml d'éthanol. On ajoute, en 15 min, 119 g (2,39 moles) d'hydrate d'hydrazine et on chauffe le mélange au reflux pendant 15h.
On refroidit le mélange à l'aide d'un bain de glace, on collecte le précipité par filtration, on le rince à l'éthanol et on le sèche sous pression réduite à 80°C pendant 2h30. On obtient ainsi 47,5 g de produit.
Point de fusion : 211°C.

### 1.2. [2-Chloro-5-méthoxy-4-(5-oxo-4,5-dihydro-1,3,4-oxadiazol-2-yl)phényl]carbamate de phénylméthyle.

Dans un réacteur de 3 l on ajoute, goutte à goutte, en l'espace d'une heure, à température ambiante et sous agitation magnétique, 461 ml (0,875 mole) d'une solution de phosgène 1,93M dans le toluène, à une suspension de 37,7 g (0,175 mole) d'hydrazide de l'acide 4-amino-5-chloro-2-méthoxybenzoïque dans 1200 ml de dioxanne.
On agite le mélange à température ambiante pendant une nuit, puis on le chauffe à 80°C pendant 1h. On chasse l'excès de phosgène par passage d'un courant d'argon à cette température pendant 2h. On ajoute alors 72 ml (0,7 mole) d'alcool benzylique et on continue à chauffer pendant 1h à 100°C. On refroidit, on concentre sous pression réduite et on triture le résidu dans l'éther isopropylique. On collecte le solide obtenu par filtration et on le sèche. On obtient ainsi 60,3 g de produit.
Point de fusion : 214°C.

### 1.3. [2-Chloro-4-[4-[1-[(1,1-diméthyléthoxy)carbonyl]pipéridin-4-yl]-5-oxo-4,5-dihydro-1,3,4-oxadiazol-2-yl]-5-méthoxyphényl]carbamate de phénylméthyle.

Dans un ballon tricol de 500 ml on introduit 15,03 g (40 mmoles) de [2-chloro-5-méthoxy-4-(5-oxo-4,5-dihydro-1,3,4-oxadiazol-2-yl)phényl]carbamate de phénylméthyle en solution dans 200 ml de tétrahydrofurane, 13,64 g (52 mmoles) de triphénylphosphine et 9,66 g de 1-[(1,1-diméthyléthoxy)carbonyl]pipéridin-4-ol, tout en agitant le mélange à 0°C. On ajoute 9,76 g (56 mmoles) d'azodicarboxylate d'éthyle, on poursuit l'agitation à 0°C pendant 1h et à température ambiante pendant 2h30.
On concentre le mélange sous pression réduite, on dissout le résidu dans de l'acétate d'éthyle, on lave la solution plusieurs fois à l'eau, on la sèche et on évapore le solvant sous pression réduite. On purifie le résidu par chromatographie sur colonne de gel de silice en éluant avec un mélange 30/70 d'acétate d'éthyle et d'hexane.
On obtient 15 g de composé sous forme de solide blanc. Point de fusion : 140°C.

### 1.4. [2-Chloro-4-(5-oxo-4-pipéridin-4-yl-4,5-dihydro-1,3,4-oxadiazol-2-yl)-5-méthoxyphényl]carbamate de phényl méthyle.

Dans un ballon tricol de 500 ml on introduit 6,52 g (12 mmoles) de [2-chloro-4-[4-[1-[(1,1-diméthyléthoxy)carbonyl]pipéridin-4-yl]-5-oxo-4,5-dihydro-1,3,4-oxadiazol-2-yl]-5-méthoxyphényl]carbamate de phénylméthyle en solution dans 140 ml de dichlorométhane et 13,64 g (120 mmoles) d'acide trifluoroacétique et on agite le mélange à température ambiante pendant une nuit.
On ajoute de la glace, puis du chloroforme, puis de l'ammoniaque aqueuse à 25%, on sépare la phase organique et on extrait la phase aqueuse quatre fois avec du chloroforme. On lave la phase organique avec une solution aqueuse saturée de chlorure de sodium, on la sèche et on évapore le solvant sous pression réduite.
On obtient 6,26 g de composé brut qu'on utilise tel quel.
Point de fusion : 180°C.

### 1.5. Bromhydrate de 5-(4-amino-5-chloro-2-méthoxyphényl)-3-pipéridin-4-yl-1,3,4-oxadiazol-2(3H)-one.

Dans un ballon de 25 ml on place 1 g (2,8 mmoles) de 2-chloro-4-(5-oxo-4-pipéridin-4-yl-4,5-dihydro-1,3,4-oxadiazol-2-yl)-5-méthoxyphényl]carbamate de phénylméthyle dissous dans 5,8 ml d'acide bromhydrique à 33% dans l'acide acétique, et on agite le mélange à température ambiante pendant 1h.
On ajoute de l'éther diéthylique et on sépare le précipité par filtration.
On obtient 0,67 g de bromhydrate.
Point de fusion : 278-280°C.
Par traitement avec de l'ammoniaque on récupère 0,52 g de base libre.

### Exemple 2 (Composé N°5).

### 5-(4-Amino-5-chloro-2-méthoxyphényl)-3-[1-(cyclohexylméthyl)pipéridin-4-yl]-1,3,4-oxadiazol-2(3H)-one.

A une solution de 1,13 g (3,48 mmoles) de 5-(4-amino-5-chloro-2-méthoxyphényl)-3-pipéridin-4-yl-1,3,4-oxadiazol-2(3*H*)-one dans 40 ml d'acétonitrile on ajoute successivement, à température ambiante, sous atmosphère d'argon et sous agitation magnétique, 2,01 ml (13,92 mmoles) de triéthylamine puis 0,92 g (5,2 mmoles) de bromure de cyclohexylméthyle dans 5 ml d'acétonitrile et on agite le mélange à 70°C pendant 2 jours.
On évapore le solvant sous pression réduite, on reprend le résidu avec du chloroforme, on lave la solution plusieurs fois à l'eau, on la sèche, on évapore le solvant sous pression réduite et on fait cristalliser le résidu dans l'acétone.
On obtient 0,7 g de solide blanc.
Point de fusion : 186,5-186,7°C.

### Exemple 3 (Composé N°9).

### 5-(4-Amino-5-chloro-2-méthoxyphényl)-3-[1-(2-phényléthyl)pipéridin-4-yl]-1,3,4-oxadiazol-2(3H)-one.

### 3.1 [2-chloro-5-méthoxy-4-[5-oxo-4-[1-(2-phényléthyl)pipéridin-4-yl]-4,5-dihydro-1,3,4-oxadiazol-2-yl]phényl]carbamate de phénylméthyle.

Dans un ballon de 100 ml on place 1,84 g (4 mmoles) de [2-chloro-4-(5-oxo-4-pipéridin-4-yl-4,5-dihydro-1,3,4-oxadiazol-2-yl)-5-méthoxyphényl]carbamate de phénylméthyle et 1,67 ml (12 mmoles) de triéthylamine en suspension dans 40 ml d'acétonitrile, on ajoute 0,96 g (5,2 mmoles) de (2-bromoéthyl)benzène dans 1 ml d'acétonitrile, on chauffe le mélange à 60°C pendant 3h, on ajoute encore 0,3 ml de (2-bromoéthyl)benzène et on chauffe à 80°C pendant une nuit.
On évapore le solvant sous pression réduite, on extrait le résidu trois fois avec du chloroforme, on lave la phase organique plusieurs fois à l'eau, on la sèche sur sulfate de sodium, on évapore le solvant sous pression réduite et on purifie le résidu par chromatographie sur colonne de gel de silice en éluant avec un mélange 80/20 d'acétate d'éthyle et d'hexane.
On obtient 2,18 g de composé pur sous forme de solide blanc. Point de fusion : 150°C.

### 3.2. 5-(4-Amino-5-chloro-2-méthoxyphényl)-3-[1-(2-phényléthyl)pipéridin-4-yl]-1,3,4-oxadiazol-2(3H)-one.

Dans un ballon de 100 ml on place 2,18 g (3,87 mmoles) de [2-chloro-5-méthoxy-4-[5-oxo-4-[1-(2-phényléthyl)pipéridin-4-yl]-4,5-dihydro-1,3,4-oxadiazol-2-yl]phényl]carbamate de phénylméthyle dissous dans une solution de 7 ml d'acide bromhydrique à 33% dans l'acide acétique et on agite le mélange à température ambiante pendant 3h.
On ajoute de l'éther diéthylique et on isole le précipité par filtration.
On obtient 1,73 g de bromhydrate.
On reprend ce dernier avec de l'eau et du chloroforme et on neutralise le mélange en ajoutant de la soude. Après séparation de la phase organique, extraction de la phase aqueuse et traitement habituel on obtient 1,44 g de composé à l'état de base libre.
Point de fusion : 184,5°C.

### Exemple 4 (Composé N°2).

### Chlorhydrate de 5-(4-amino-5-chloro-2-méthoxyphényl)-3-(1-méthylpipéridin-4-yl)-1,3,4-oxadiazol-2(3H)-one.

### 4.1. [2-Chloro-4-[4-(1-méthylpipéridin-4-yl)-5-oxo-4,5-dihydro-1,3,4-oxadiazol-2-yl]-5-méthoxyphényl]carbamate de phénylméthyle.

Dans un ballon de 2,5 l on place 7,5 g de [2-chloro-5-méthoxy-4-(5-oxo-4,5-dihydro-1,3,4-oxadiazol-2-yl)phényl]carbamate de phénylméthyle en suspension dans 150 ml de tétrahydrofurane, 6,82 g (26 mmoles) de triphénylphosphine et 2,3 g (20 mmoles) de 1-méthylpipéridin-4-ol, on ajoute 4,39 g (28 mmoles) d'azodicarboxylate d'éthyle à 0°C et sous agitation magnétique, et on maintient l'agitation pendant 20h.
On concentre le mélange sous pression réduite, on reprend le résidu avec de l'acétone, on refroidit à 0°C, et on isole le précipité par filtration.
On obtient 5,02 g de composé sous forme de solide blanc.
Point de fusion : 142°C.

### 4.2. 5-(4-Amino-5-chloro-2-méthoxyphényl)-3-(1-méthylpipéridin-4-yl)-1,3,4-oxadiazol-2(3H)-one.

Dans un ballon de 100 ml on place 2 g (4,23 mmoles) de [2-chloro-4-[4-(1-méthylpipéridin-4-yl)-5-oxo-4,5-dihydro-1,3,4-oxadiazol-2-yl]-5-méthoxyphényl]carbamate de phénylméthyle dissous dans 20 ml d'acide acétique, on ajoute lentement 20 ml d'acide bromhydrique à 33% dans l'acide acétique et on agite le mélange à température ambiante pendant 18h.
On ajoute de l'éther diéthylique et on isole le solide par filtration.
On obtient 2 g de bromhydrate.
On le dissout dans 30 ml d'eau, on neutralise la solution avec de la soude, on sépare le précipité par filtration, on le lave à l'eau et on le sèche sous pression réduite.
On obtient 1,05 g de composé sous forme de base libre.
Point de fusion : 162°C.
Par traitement avec l'acide chlorhydrique dans l'éthanol on obtient le chlorhydrate.
Point de fusion : 212-218°C.

### Exemple 5 (Composé N°19).

### Chlorhydrate de 5-[4-amino-5-chloro-2-(cyclopropylméthoxy)phényl]-3-(1-butylpipéridin-4-yl)-1,3,4-oxadiazol-2(3H)-one.

### 5.1. 4-Amino-5-chloro-2-(cyclopropylméthoxy)benzoate de méthyle.

Dans un ballon tricol de 1 l on introduit 29,1 g (0,120 mole) d'acide 4-amino-5-chloro-2-(cyclopropylméthoxy)benzoïque et 340 ml de méthanol, on refroidit la solution à -40°C, on ajoute, goutte à goutte, 44 ml (0,602 mole) de chlorure de thionyle, et on chauffe le mélange au reflux pendant 1h30. On le refroidit, on évapore le solvant, on reprend le résidu avec de l'eau et une solution aqueuse de carbonate de sodium, on extrait au dichlorométhane et on purifie le produit brut par chromatographie sur colonne de gel de silice en éluant avec un mélange de n-heptane et d'acétate d'éthyle de 90/10 à 80/20.
On obtient 8,3 g de composé sous forme de solide jaune pâle. Point de fusion : 115°C.

### 5.2. Hydrazide de l'acide 4-amino-5-chloro-2-cyclopropyl_ méthoxybenzoïque.

Dans un ballon de 250 ml on introduit 6,0 g (23,5 mmoles) de 4-amino-5-chloro-2-(cyclopropylméthoxy)benzoate de méthyle et 54 ml d'éthanol, on ajoute, à 40°C, 118g (235 mmoles) d'hydrate d'hydrazine et on chauffe le mélange au reflux pendant 18h.
On le refroidit avec un bain de glace, on isole le précipité par filtration, on le rince à l'éthanol et on le sèche sous pression réduite à 70°C pendant 4h.
On obtient 4,7 g de composé.
Point de fusion : 172°C.

### 5.3. 5-[4-Amino-5-chloro-2-(cyclopropylméthoxy)phényl]-1,3,4-oxadiazol-2(3H)-one.

Dans un ballon de 100 ml on introduit 2,0 g (7,8 mmoles) d'hydrazide de l'acide 4-amino-5-chloro-2-(cyclopropylméthoxy)benzoïque, 17 ml de toluène et 1,9 ml (8,6 mmoles) de chloroformiate de phényle et on chauffe le mélange au reflux pendant 4h.
On le refroidit à température ambiante, on ajoute 2,5 ml (16,4 mmoles) de triéthylamine, on chauffe au reflux pendant 3h, on refroidit à température ambiante, on ajoute de l'eau, on extrait avec du chloroforme. Après traitement habituel et purification par chromatographie sur colonne de gel de silice en éluant avec un mélange 98/2/0,2 de chloroforme, méthanol et ammoniaque, on obtient 0,80 g de solide blanc.
Point de fusion : 153-154°C.

### 5.4. Chlorhydrate de 5-[4-amino-5-chloro-2-(cyclopropylméthoxy)phényl]-3-(1-butylpipéridin-4-yl)-1,3,4-oxadiazol-2(3H)-one.

A partir de 5-[4-amino-5-chloro-2-(cyclopropylméthoxy)phényl]-1,3,4-oxadiazol-2(3*H*)-one et de 1-butylpipéridin-4-ol, en opérant selon la méthode décrite dans l'exemple 4.1, on obtient le composé final à l'état de base et, après traitement avec une solution d'acide chlorhydrique dans l'éthanol et recristallisation dans l'éthanol, on obtient le chlorhydrate.
Point de fusion : 237-238°C.

### Exemple 6 (Composé N°28).

### Bromhydrate de 5-(8-Amino-7-chloro-2,3-dihydro-1,4-benzodioxin-5-yl)-3-pipéridin-4-yl-1,3,4-oxadiazol-2(3H)-one.

### 6.1. 8-Amino-2,3-dihydro-1,4-benzodioxine-5-carboxylate d'éthyle.

Dans un ballon tricol de 2 l contenant 772 ml d'éthanol refroidi à -40°C, sous agitation, on introduit lentement 23,5 g (0,198 mole) de chlorure de thionyle, on maintient l'agitation à cette température pendant 1h, on ajoute lentement, en 15 min, 38,6 g (0,198 mole) d'acide 8-amino-2,3-dihydro-1,4-benzodioxine-5-carboxylique en solution dans 100 ml d'éthanol, et on laisse le mélange revenir à température ambiante pendant une nuit.
On le chauffe au reflux pendant 4h, on évapore le solvant sous pression réduite, on reprend le résidu avec de l'eau et du carbonate de sodium et on l'extrait avec du chloroforme. Après lavage, séchage et évaporation de la phase organique on obtient 34,06 g d'ester sous forme de solide blanc.
Point de fusion : 112°C.

### 6.2. 8-Amino-7-chloro-2,3-dihydro-1,4-benzodioxine-5-carboxylate d'éthyle.

Dans un ballon de 1 1 on introduit 37 g (0,165 mole) de 8-amino-2,3-dihydro-1,4-benzodioxine-5-carboxylate d'éthyle en solution dans 370 ml de dioxane, on ajoute, à température ambiante et sous agitation magnétique, 23,2 g (0,174 mole) de *N*-chlorosuccinimide et on maintient l'agitation pendant une nuit.
On dilue le mélange à l'eau, on l'extrait à l'acétate d'éthyle et, après traitement habituel de la phase organique, on obtient 42 g de composé qu'on recristallise dans un mélange d'éther diéthylique et d'éther diisopropylique.
Point de fusion : 105-106°C.

### 6.3. Hydrazide de l'acide 8-amino-7-chloro-2,3-dihydro-1,4-benzodioxine-5-carboxylique.

Dans un réacteur de 1 l on introduit 38,4 g (0,149 mole) de 8-amino-7-chloro-2,3-dihydro-1,4-benzodioxine-5-carboxylate d'éthyle en suspension dans 150 ml d'éthanol, on ajoute, en 15 min, 149 g (2,98 mole) d'hydrate d'hydrazine et on chauffe le mélange au reflux pendant 1h.
On le refroidit à l'aide d'un bain de glace,, on recueille le précipité par filtration, on le lave à l'éthanol et on le sèche sous pression réduite.
On obtient 33 g de composé.
Point de fusion : 227-231°C.

### 6.4. [6-chloro-8-(5-oxo-4,5-dihydro-1,3,4-oxadiazol-2-yl)-2,3-dihydro-1,4-benzodioxin-5-yl]carbamate de phénylméthyle.

Dans un réacteur de 1 l, à température ambiante et sous agitation magnétique, on introduit 32,6 g d'hydrazide de l'acide 8-amino-7-chloro-2,3-dihydro-1,4-benzodioxine-5-carboxylique et 330 ml de dioxane, on ajoute à cette suspension, goutte à goutte, et en l'espace d'une heure et demie, 310 ml (0,4 mole) d'une solution de phosgène 0,193M dans le toluène, on agite le mélange à température ambiante pendant une nuit et on le chauffe au reflux pendant 5h.
On chasse l'excès de phosgène à cette température par passage d'un courant d'argon pendant 2h, on refroidit le mélange, on le concentre sous pression réduite, on reprend le résidu avec 200 ml d'alcool benzylique, on le chauffe à 100°C pendant une nuit, on refroidit le mélange, on le concentre sous pression réduite et on triture le résidu dans l'éther diisopropylique. Après filtration et séchage on obtient 52,6 g de composé.
Point de fusion : 230°C.

### 6.5. [6-Chloro-8-[4-[1-[(1,1-diméthyléthoxy)carbonyl]pipéridin-4-yl]-5-oxo-4,5-dihydro-1,3,4-oxadiazol-2-yl]-2,3-dihydro-1,4-benzodioxin-5-yl]carbamate de phénylméthyle.

Dans un ballon tricol de 250 ml, à 0°C et sous agitation magnétique, on introduit 8,07 g (20 mmoles) de [6-chloro-8-(5-oxo-4,5-dihydro-1,3,4-oxadiazol-2-yl)-2,3-dihydro-1,4-benzodioxin-5-yl]carbamate de phénylméthyle, 160 ml de tétrahydrofurane, 6,83 g (26 mmoles) de triphénylphosphine, 4,83 g (24 mmoles) de 1-[(1,1-diméthyléthoxy)carbonyl]pipéridin-4-ol, puis 4,52 g (26 mmoles) d'azodicarboxylate d'éthyle. Après 1h d'agitation à 0°C et 2h30 d'agitation à température ambiante, on concentre le mélange sous pression réduite, et on recristallise le résidu une première fois dans l'éther diéthylique et une seconde fois dans l'acétate d'éthyle. On obtient 5,5 g de composé sous forme de solide blanc.
Point de fusion : 206°C.

### 6.6. [6-Chloro-8-(5-oxo-4-pipéridin-4-yl-4,5-dihydro-1,3,4-oxadiazol-2-yl)-2,3-dihydro-1,4-benzodioxin-5-yl]carbamate de phénylméthyle.

Dans un ballon tricol de 250 ml on introduit 5,3 g (9 mmoles) de [6-chloro-8-[4-[1-[(1,1-diméthyléthoxy)carbonyl]pipéridin-4-yl]-5-oxo-4,5-dihydro-1,3,4-oxadiazol-2-yl]-2,3-dihydro-1,4-benzodioxin-5-yl]carbamate de phénylméthyle, 100 ml de dichlorométhane et 10,3 g (90 mmoles) d'acide trifluoroacétique, et on agite le mélange à température ambiante pendant une nuit.
On concentre le mélange sous pression réduite, on triture le résidu dans l'acétone, on le collecte par filtration, on le lave à l'éther diéthylique, on le traite par addition lente de 17 ml d'ammoniaque aqueuse à 25%, et on l'extrait quatre fois avec du chloroforme. Après lavage avec de l'eau puis avec une solution saturée de chlorure de sodium, séchage et évaporation du solvant, on obtient 4,4 g de composé qu'on utilise tel quel dans l'étape suivante.
Point de fusion : 128-130°C.

### 6.7. 5-(8-Amino-7-chloro-2,3-dihydro-1,4-benzodioxin-5-yl)-3-pipéridin-4-yl-1,3,4-oxadiazol-2(3H)-one.

Dans un ballon de 50 ml on introduit 3,68 g (27,5 mmoles) de [6-chloro-8-(5-oxo-4-pipéridin-4-yl-4,5-dihydro-1,3,4-oxadiazol-2-yl)-2,3-dihydro-1,4-benzodioxin-5-yl]carbamate de phénylméthyle et 35 ml d'acide acétique, on ajoute 11 ml d'acide bromhydrique à 33% dans l'acide acétique, et on agite le mélange à température ambiante pendant 22h.
On ajoute de l'éther diéthylique au précipité qui s'est formé, et on le recueille par filtration. On obtient 4 g de bromhydrate.
Point de fusion >260°C.
Par traitement avec de la soude on récupère le composé sous forme de base libre.
Point de fusion : 213-215°C.

### Exemple 7 (Composé N°37)

### 5-(8-Amino-7-chloro-2,3-dihydro-1,4-benzodioxin-5-yl)-3-[1-[[4-(trifluorométhyl)phényl]méthyl]pipéridin-4-yl]-1,3,4-oxadiazol-2(3H)-one.

A une solution de 1 g (2,84 mmoles) de 5-(8-amino-7-chloro-2,3-dihydro-1,4-benzodioxin-5-yl)-3-pipéridin-4-yl-1,3,4-oxadiazol-2(3H)-one dans 60 ml d'acétonitrile, à température ambiante, sous agitation magnétique et sous atmosphère d'argon, on ajoute successivement 1,58 ml (11,38 mmoles) de triéthylamine et 0,88 g (5,68 mmoles) de bromure de (4-trifluorométhyl)benzyle en solution dans 5 ml d'acétonitrile, et on agite le mélange pendant 2h.
On évapore le solvant sous pression réduite, on reprend le résidu avec du chloroforme, on lave la solution plusieurs fois à l'eau, on la sèche et on évapore le solvant sous pression réduite.
On fait cristalliser le résidu dans l'acétone et on obtient 1,14 g de solide blanc.
Point de fusion : 198°C.

### Exemple 8 (Composé N°32).

### 5-(8-Amino-7-chloro-2,3-dihydro-1,4-benzodioxin-5-yl)-3-[1-(4,4,4-trifluorobutyl)pipéridin-4-yl]-1,3,4-oxadiazol-2(3H)-one.

### 8.1. [6-Chloro-8-[5-oxo-4-[1-(4,4,4-trifluorobutyl)pipéridin-4-yl]-4,5-dihydro-1,3,4-oxadiazol-2-yl]-2,3-dihydro-1,4-benzodioxin-5-yl]carbamate de phénylméthyle.

Dans un ballon de 100 ml on introduit 2 g (4,1 mmoles) de [6-chloro-8-(5-oxo-4-pipéridin-4-yl-4,5-dihydro-1,3,4-oxadiazol-2-yl)-2,3-dihydro-1,4-benzodioxin-5-yl]carbamate de phénylméthyle, 40 ml d'acétonitrile et 2,3 ml (16 mmoles) de triéthylamine, on ajoute 1,5 g (6,67 mmoles) de bromure de 4,4,4-trifluorobutyle dans 1 ml d'acétonitrile, et on chauffe le mélange à 80°C pendant une nuit.
On évapore le solvant sous pression réduite, on extrait le résidu trois fois avec du chloroforme, on lave, sèche et évapore le phase organique.
Après purification du résidu par chromatographie sur colonne de gel de silice en éluant avec un mélange 97/3/0,3 de dichlorométhane, de méthanol et d'ammoniaque on obtient 2,4 g de composé sous forme de solide blanc.
Point de fusion : 158°C.

### 8.2. 5-(8-Amino-7-chloro-2,3-dihydro-1,4-benzodioxin-5-yl)-3-[1-(4,4,4-trifluorobutyl)pipéridin-4-yl]-1,3,4-oxadiazol-2(3H)-one.

Dans un ballon de 100 ml on introduit 1,72 g (2,88 mmoles) de [6-chloro-8-[5-oxo-4-[1-(4,4,4-trifluorobutyl)pipéridin-4-yl]-4,5-dihydro-1,3,4-oxadiazol-2-yl]-2,3-dihydro-1,4-benzodioxin-5-yl]carbamate de phénylméthyle, 17 ml d'acide acétique, on ajoute 5 ml d'acide bromhydrique à 33% dans l'acide acétique, et on agite à température ambiante pendant 7h. On ajoute de l'éther diéthylique au précipité qui s'est formé et, par filtration, on recueille 1,8 g de bromhydrate.
On le reprend avec de l'eau et du chloroforme, on ajoute de la soude pour libérer la base et, après traitement habituel de la phase organique on obtient 1,19 g de composé.
Point de fusion : 188°C.

### Exemple 9 (Composé N°29).

### Bromhydrate de 5-(8-amino-7-chloro-2,3-dihydro-1,4-benzodioxin-5-yl)-3-(1-méthylpipéridin-4-yl)-1,3,4-oxadiazol-2(3H)-one.

### 9.1. [6-Chloro-8-[4-(1-méthylpipéridin-4-yl)-5-oxo-4,5-dihydro-1,3,4-oxadiazol-2-yl]-2,3-dihydro-1,4-benzodioxin-5-yl]carbamate de phénylméthyle.

Dans un ballon de 250 ml on introduit 6,27 g (15,53 mmoles) de [6-chloro-8-(5-oxo-4,5-dihydro-1,3,4-oxadiazol-2-yl)-2,3-dihydro-1,4-benzodioxin-5-yl]carbamate de phénylméthyle en suspension dans 80 ml de tétrahydrofurane, 6,12 g (23,3 mmoles) de triphénylphosphine, 2,24 g (19,4 mmoles) de 1-méthylpipéridin-4-ol, et, à 0°C et sous agitation, on ajoute lentement 4,06 ml (23,3 mmoles) d'azodicarboxylate d'éthyle, et on agite le mélange pendant 48h.
On concentre le mélange sous pression réduite, on le reprend avec de l'eau, on ajoute 1,6 ml d'acide chlorhydrique à 37% puis 60 ml d'acétate d'éthyle, on agite le mélange pendant 1h, puis on l'extrait quatre fois à l'acétate d'éthyle. On évapore le solvant sous pression réduite, on traite le résidu avec de l'ammoniaque jusqu'à pH=10, et on collecte le précipité par filtration.
On obtient 3,16 g de composé sous forme de solide blanc.
Point de fusion : 177°C.

### 9.2. Bromhydrate de 5-(8-amino-7-chloro-2,3-dihydro-1,4-benzodioxin-5-yl)-3-(1-méthylpipéridin-4-yl)-1,3,4-oxadiazol-2(3H)-one.

Dans un ballon de 100 ml on introduit 1,71 g (3,41 mmoles) de [6-chloro-8-[4-(1-méthylpipéridin-4-yl)-5-oxo-4,5-dihydro-1,3,4-oxadiazol-2-yl]-2,3-dihydro-1,4-benzodioxin-5-yl]carbamate de phénylméthyle en solution dans 30 ml d'acide acétique, on ajoute lentement 3 ml d'acide bromhydrique à 33% dans l'acide acétique et on agite le mélange pendant 5h. On ajoute de l'éther diéthylique au précipité qui s'est formé, et on recueille ce dernier par filtration.
On obtient 1,72 g de bromhydrate.
Point de fusion : 248°C.

### Exemple 10 (Composé N°31).

### Chlorhydrate de 5-(8-amino-7-chloro-2,3-dihydro-1,4-benzodioxin-5-yl)-3-(1-butylpipéridin-4-yl)-1,3,4-oxadiazol-2(3H)-one.

### 10.1. [8-[4-(1-Butylpipéridin-4-yl)-5-oxo-4,5-dihydro-1,3,4-oxadiazol-2-yl]-6-chloro-2,3-dihydro-1,4-benzodioxin-5-yl]carbamate de phénylméthyle.

On opère comme décrit dans l'exemple 9.1, à partir de [6-chloro-8-(5-oxo-4,5-dihydro-1,3,4-oxadiazol-2-yl)-2,3-dihydro-1,4-benzodioxin-5-yl]carbamate de phénylméthyle et de 1-butylpipéridin-4-ol.

### 10.2. Chlorhydrate de 5-(8-amino-7-chloro-2,3-dihydro-1,4-benzodioxin-5-yl)-3-(1-butylpipéridin-4-yl)-1,3,4-oxadiazol-2(3H)-one.

On opère comme décrit dans l'exemple 9.2, à partir de [8-[4-(1-butylpipéridin-4-yl)-5-oxo-4,5-dihydro-1,3,4-oxadiazol-2-yl]-6-chloro-2,3-dihydro-1,4-benzodioxin-5-yl]carbamate de phénylméthyle et d'acide bromhydrique, et on forme le chlorhydrate par traitement à l'acide chlorhydrique dans l'éthanol.
Point de fusion : 280-283°C.

### Exemple 11 (Composé N°62).

### Chlorhydrate de 5-(5-chloro-2,3-dihydrobenzofuran-7-yl)-3-pipéridin-4-yl-1,3,4-oxadiazol-2(3H)-one.

### 11.1. Hydrazide de l'acide 5-chloro-2,3-dihydrobenzofurane-7-carboxylique.

A 25,14 g (0,118 mole) de 5-chloro-2,3-dihydrobenzofurane-7-carboxylate de méthyle en suspension dans 300 ml de méthanol on ajoute 57,3 ml (1,18 mole) d'hydrate d'hydrazine et on chauffe le mélange au reflux pendant 4 h.
On le refroidit à l'aide d'un bain de glace, on recueille le précipité par filtration, on le lave à l'éthanol et on le sèche sous pression réduite.
On obtient 24,34 g de composé.
Point de fusion : 182°C.

### 11.2. 5-(5-Chloro-2,3-dihydrobenzofuran-7-yl)-1,3,4-oxadiazol-2(3H)-one.

Dans un réacteur de 1 l, à température ambiante et sous agitation magnétique, on introduit 24,34 g (0,115 mole) d'hydrazide de l'acide 5-chloro-2,3-dihydrobenzofurane-7-carboxylique et 500 ml de dioxanne, on ajoute, au moyen d'une ampoule à brome, 178 ml (0,343 mole) de phosgène 0,193 M dans le toluène, on agite le mélange à température ambiante pendant 24 h, puis au reflux pendant 4 h pour chasser l'excès de phosgène.
On évapore le solvant sous pression réduite, on reprend le résidu avec de l'éther diéthylique, on le collecte par filtration et on le sèche.
On obtient 27 g de composé.
Point de fusion : 270°C.

### 11.3. 5-(5-Chloro-2,3-dihydrobenzofuran-7-yl)-3-[1-[(1,1-diméthyléthoxy)carbonyl]pipéridin-4-yl]-1,3,4-oxadiazol-2(3H)-one.

Dans un ballon tricol de 500 ml, refroidi à 0°C et placé sous agitation magnétique, on introduit 20 g (0,08 mole) de 5-(5-chloro-2,3-dihydrobenzofuran-7-yl)-1,3,4-oxadiazol-2(3*H*)-one en suspension dans 250 ml de tétrahydrofurane, on ajoute 10,06 g (0,05 mole) de 1-[(1,1-diméthyléthoxy)carbonyl]pipéridin-4-ol, 18,36 g de triphénylphosphine, et 14,81 g (0,085 mole) d'azodicarboxylate d'éthyle, et on agite le mélange à température ambiante pendant 4 h.
On concentre le mélange sous pression réduite et on recristallise le résidu dans un mélange de dichlorométhane et d'éther diéthylique.
On obtient 14,7 g de solide beige.
Point de fusion : 203°C.

### 11.4. Chlorhydrate de 5-(5-chloro-2,3-dihydrobenzofuran-7-yl)-3-pipéridin-4-yl-1,3,4-oxadiazol-2(3H)-one.

Dans un ballon de 500 ml on dissout 14,7 g (0,035 mole) de 5-(5-chloro-2,3-dihydrobenzofuran-7-yl)-3-[1-[(1,1-diméthyléthoxy)carbonyl]pipéridin-4-yl]-1,3,4-oxadiazol-2(3H)-one dans 150 ml de dichlorométhane, on ajoute, à 0°C, 26,8 ml d'acide trifluoroacétique et on agite le mélange à température ambiante pendant 3 h.
On ajoute 200 ml d'eau et 47 ml de soude à 30% dans 300 ml d'eau, on extrait le mélange avec du chloroforme, on sèche la phase organique et on évapore le solvant sous pression réduite.
On obtient 10,8 g de base sous forme d'un solide blanc. Point de fusion : 180°C.
Par traitement de 5 g de base avec une solution d'acide chlorhydrique gazeux dans l'éthanol on obtient 3,5 g de chlorhydrate.
Point de fusion : >260°C.

### Exemple 12 (Composé N°67).

### Chlorhydrate de 5-(5-chloro-2,3-dihydrobenzofuran-7-yl)-3-[1-(2-phényléthyl)pipéridin-4-yl]-1,3,4-oxadiazol-2(3H)-one.

Dans un ballon tricol de 250 ml on introduit 2,5 g (7,77 mmoles) de 5-(5-chloro-2, 3-dihydrobenzofuran-7-yl)-3-pipéridin-4-yl-1,3,4-oxadiazol-2(3H)-one en solution dans 50 ml de butan-2-one, on ajoute 2,87 g (15,5 mmoles) de bromure de phényléthyle puis 2,36 g (23,3 mmoles) de triéthylamine et on agite le mélange au reflux pendant 20 h. On collecte le précipité formé par filtration, on évapore le filtrat sous pression réduite, on reprend le résidu avec de l'eau, on l'extrait deux fois avec du chloroforme, et on évapore la phase organique sous pression réduite.
On dissout le résidu dans une solution d'acide chlorhydrique gazeux dans l'éthanol, on ajoute de l'éther diéthylique, on recueille le précipité par filtration, et on le recristallise dans l'éthanol. On obtient 2,0 g de chlorhydrate.
Point de fusion : 255-257°C.

### Exemple 13 (Composé N°65).

### 5-(5-Chloro-2,3-dihydrobenzofuran-7-yl)-3-(1-butylpipéridin-4-yl)-1,3,4-oxadiazol-2(3H)-one.

Dans un ballon tricol de 250 ml, refroidi à 0°C et placé sous agitation magnétique, on introduit 2,38 g (0,01 mole) de 5-(5-chloro-2,3-dihydrobenzofuran-7-yl)-1,3,4-oxadiazol-2(3*H*)-one en suspension dans 80 ml de tétrahydrofurane, on ajoute 1,57 g (0,01 mole) de 1-butylpipéridin-4-ol, 3,41 g (0,013 mole) de triphénylphosphine puis 2,44 g (0,014 mole) d'azodicarboxylate d'éthyle, et on agite le mélange à température ambiante pendant 3 h puis à 40°C pendant 3 h.
On évapore le solvant sous pression réduite, on reprend le résidu avec de l'eau et on l'extrait cinq fois avec de l'éther diéthylique.
On sèche la phase organique sur sulfate de magnésium, on la filtre, on évapore le solvant sous pression réduite et on purifie le résidu par chromatographie sur colonne de gel de silice en éluant avec un mélange 80/20 d'acétate d'éthyle et d'heptane.
On obtient 3 g de composé.
Point de fusion : 133,8-134°C.

### Exemple 14 (Composé N°71).

### Chlorhydrate de 5-(6-chloro-3,4-dihydro-2H-benzopyran-8-yl)-3-pipéridin-4-yl-1,3,4-oxadiazol-2(3H)-one.

### 14.1. Hydrazide de l'acide 6-chloro-3,4-dihydro-2H-benzopyrane-8-carboxylique.

A 34 g (0,15 mole) de 6-chloro-3,4-dihydro-2*H*-benzopyrane-8-carboxylate de méthyle en solution dans 250 ml d'éthanol on ajoute 72,8 ml (1,5 mole) d'hydrate d'hydrazine, et on chauffe le mélange au reflux pendant 8 h.
On le refroidit à l'aide d'un bain de glace, on recueille le précipité par filtration, on le lave à l'éthanol et on le sèche sous pression réduite.
On obtient 31 g de composé.
Point de fusion : 149°C.

### 14.2. 5-(6-Chloro-3,4-dihydro-2H-benzopyran-6-yl)-1,3,4-oxadiazol-2(3H)-one.

Dans un réacteur de 1 l, à température ambiante et sous agitation magnétique, on introduit 31 g (0,137 mole) d'hydrazide de l'acide 6-chloro-3,4-dihydro-2H-benzopyrane-8-carboxylique et 500 ml de dioxane, on ajoute, au moyen d'une ampoule à brome, 212,7 ml (0,411 mole) de phosgène 0,139 M dans le toluène, et on chauffe le mélange au reflux pendant 2 h.
On recueille le précipité par filtration, et on le sèche.
On obtient 26 g de composé.
Point de fusion : 246°C.

### 14.3. Chlorhydrate de 5-(6-chloro-3,4-dihydro-2H-benzopyran-8-yl)-3-pipéridin-4-yl-1,3,4-oxadiazol-2(3H)-one.

Dans un ballon tricol de 500 ml, refroidi à 0°C et placé sous agitation magnétique, on introduit 20 g (0,08 mole) de 5-(6-chloro-3,4-dihydro-2*H*-benzopyran-8-yl)-1,3,4-oxadiazol-2(3*H*)-one en suspension dans 300 ml de tétrahydrofurane, on ajoute 15,94 g (0,08 mole) de 1-[(1,1-diméthyléthoxy)carbonyl]pipéridin-4-ol, 35,36 g (0,134 mole) de triphénylphosphine, et 21,1 ml (0,134 mole) d'azodicarboxylate d'éthyle, et on agite le mélange à température ambiante pendant 4 h. On concentre le mélange sous pression réduite, on dissout le résidu dans 250 ml de dichlorométhane, on refroidit la solution à 0°C, on ajoute 100 ml d'acide trifluoroacétique et on agite le mélange à température ambiante pendant 2 h.
On le concentre sous pression réduite, on ajoute 100 ml d'une solution 1 N d'acide chlorhydrique aqueux, on recueille le précipité par filtration et on le sèche.
On obtient 19 g de chlorhydrate.
Point de fusion : 297°C.

### Exemple 15 (Composé N°77).

### Chlorhydrate de 5-(6-chloro-3,4-dihydro-2H-benzopyran-8-yl)-3-[1-[4-[4-(diméthylamino)pipéridin-1-yl]-4-oxobutyl]pipéridin-4-yl]-1,3,4-oxadiazol-2(3H)-one.

Dans un ballon tricol de 250 ml on introduit 1 g (2,68 mmoles) de chlorhydrate de 5-(6-chloro-3,4-dihydro-2*H*-benzopyran-8-yl)-3-pipéridin-4-yl-1,3,4-oxadiazol-2(3*H*)-one en solution dans 50 ml d'acétonitrile, on ajoute 1,24 ml (5,36 mmoles) de 1-(4-chloro-1-oxobutyl)-*N,N*-diméthylpipéridin-4-amine et 1,12 ml (8 mmoles) de triéthylamine, et on agite le mélange à température ambiante pendant une nuit. On évapore le mélange sous pression réduite, on reprend le résidu avec de l'eau et on l'extrait trois fois avec du chloroforme. On sèche la phase organique sur sulfate de magnésium, on la filtre, on évapore le solvant sous pression réduite, et on purifie le résidu par chromatographie sur colonne de gel de silice en éluant avec des mélanges 98/2, 95/5 puis 90/10 de dichlorométhane et de méthanol.
On obtient un solide blanc qu'on traite dans une solution d'acide chlorhydrique gazeux dans l'éthanol et, après recristallisation dans l'éthanol, on isole finalement 0,22 g de chlorhydrate.
Point de fusion : 193°C.

### Exemple 16 (Composé N°74).

### Chlorhydrate de 5-(6-chloro-3,4-dihydro-2H-benzopyran-8-yl)-3-[1-(1-méthyléthyl)pipéridin-4-yl]-1,3,4-oxadiazol-2(3H)-one.

A partir de 0,5 g (1,34 mmole) de chlorhydrate de 5-(6-chloro-3,4-dihydro-2*H*-benzopyran-8-yl)-3-pipéridin-4-yl-1,3,4-oxadiazol-2(3*H*)-one et de 0,378 g (1,34 mmole) de 1-bromo-1-méthyléthane, et en opérant comme décrit dans l'exemple 15, on obtient 0,33 g de composé.
Point de fusion : 241°C.

### Exemple 17 (Composé N°81)

### Chlorhydrate de 5-(6-chloro-3,4-dihydro-2H-benzopyran-8-yl)-3-[1-(2,3-dihydro-1H-indén-2-yl)pipéridin-4-yl]-1,3,4-oxadiazol-2(3H)-one.

A une solution de 1,13 g (8,60 mmoles) d'indan-2-one dans 15 ml de méthanol contenant 0,169 ml d'acide acétique on ajoute 1,0 g (2,68 mmoles) de chlorhydrate de 5-(6-chloro-3,4-dihydro-2*H*-benzopyran-8-yl)-3-pipéridin-4-yl-1,3,4-oxadiazol-2(3*H*)-one et, à une température de 5°C, on ajoute 0,709 g (1,13 mmole) de cyanoborohydrure de sodium et on agite le mélange pendant 18 h.
On ajoute 15 ml d'acide chlorhydrique aqueux et, après 30 min d'agitation, on neutralise le mélange avec de la soude aqueuse 2M.
On extrait le mélange au dichlorométhane, on sépare la phase organique, on la sèche et on évapore le solvant sous pression réduite. On purifie le résidu par chromatographie sur couche mince préparative en éluant avec un mélange 98/2 de dichlorométhane et de méthanol.
On obtient un solide blanc dont on prépare le chlorhydrate de façon habituelle. On isole 0,8 g de sel.
Point de fusion : 283-284°C.

### Exemple 18 (Composé N°75)

### Fumarate (2:1) de 5-(6-chloro-3,4-dihydro-2H-benzopyran-8-yl)-3-[1-[5-[4-(diméthylamino)pipéridin-1-yl]-5-oxopentyl]pipéridin-4-yl]-1,3,4-oxadiazol-2(3H)-one.

On chauffe une suspension de 2,0 g (5,37 mmoles) de chlorhydrate de 5-(6-chloro-3,4-dihydro-2*H*-benzopyran-8-yl)-3-pipéridin-4-yl-1,3,4-oxadiazol-2(3*H*)-one dans 75 ml d'acétonitrile contenant 2,24 ml (16 mmoles) de triéthylamine et 1,44 g (5,37 mmoles) de 1-(5-chloro-1-oxopentyl)-*N,N*-diméthylpipéridin-4-amine au reflux pendant 2 h.
On ajoute 2,88 g (10,74 mmoles) de 1-(5-chloro-1-oxopentyl)-*N,N*-diméthylpipéridin-4-amine supplémentaire et on continue de chauffer pendant 18 h.
On évapore le solvant sous pression réduite, on reprend le résidu avec de l'eau et on l'extrait au chloroforme. Après séchage de la phase organique on purifie le résidu par chromatographie sur colonne de gel de silice en éluant avec un mélange 98/2 à 90/10 de dichlorométhane et de méthanol.
On obtient 0,4 g de produit à l'état de base, dont on prépare le difumarate de façon habituelle.
Point de fusion : 127°C.

Le tableau qui suit illustre les structures chimiques et les propriétés physiques de quelques composés selon l'invention. Dans les colonnes R₁ et R₂, cC₃H₅ désigne un groupe cyclopropyle, cC₆H₁₁ un groupe cyclohexyle, C₆H₅ un groupe phényle, C₆H₄-n-X un groupe phényle substitué par X en position n, C₆H₃-m,n-X₂ un groupe phényle disubstitué par X en positions m et n, 1-C₉H₉ un groupe 2,3-dihydro-1*H*-indén-1-yle, 2-C₉H₉ un groupe 2,3-dihydro-1*H*-indén-2-yle, NC₅H₁₀ un groupe pipéridin-1-yle et NC₅H₉-4-N(CH₃)₂ un groupe 4-(diméthylamino)pipéridin-1-yle.
Dans la colonne Sel, - désigne la base, HBr désigne un bromhydrate, HCl un chlorhydrate, 2HCl un chlorhydrate (2:1), fum. un fumarate, 2fum. un fumarate (2:1) et tar. un tartrate.
Dans la colonne F (°C), (d) désigne un point de fusion avec décomposition.

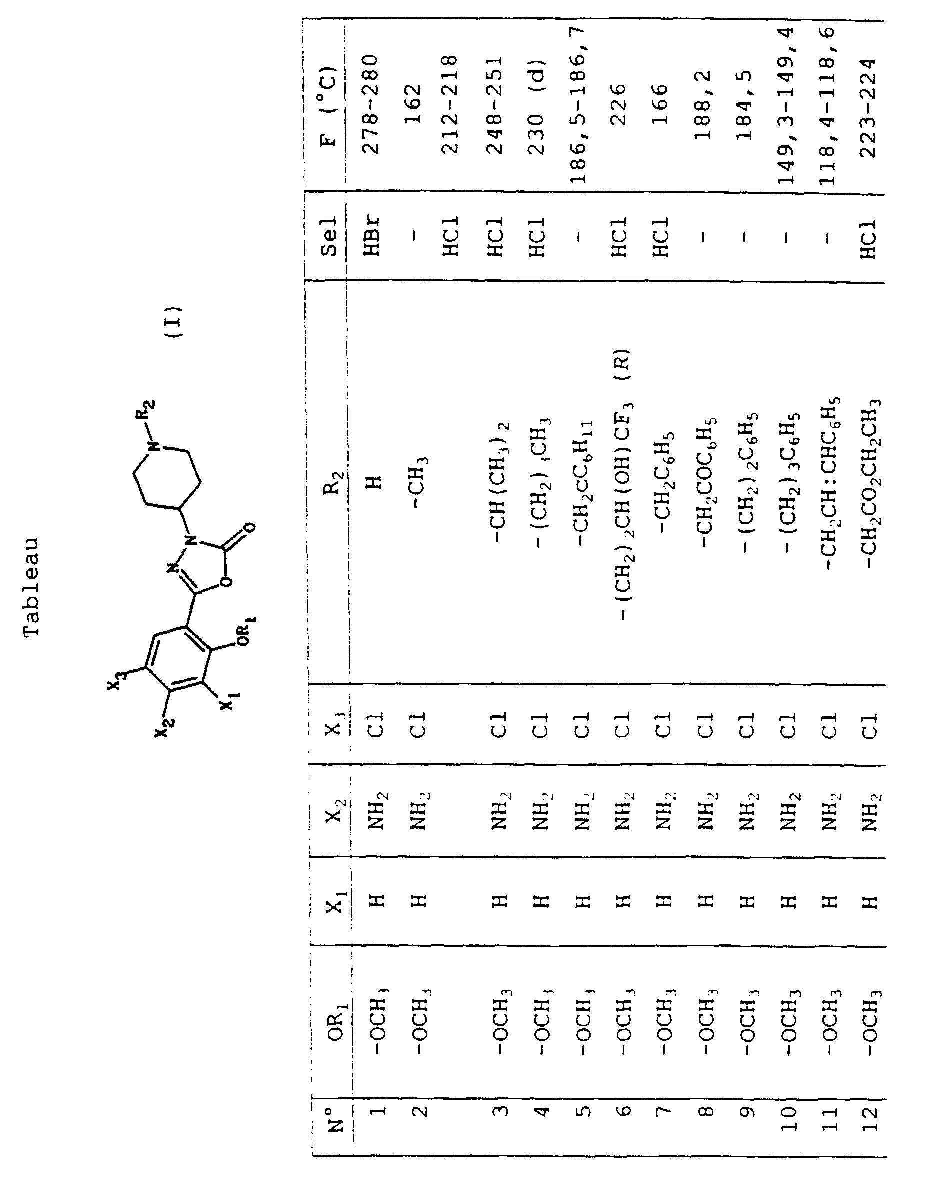

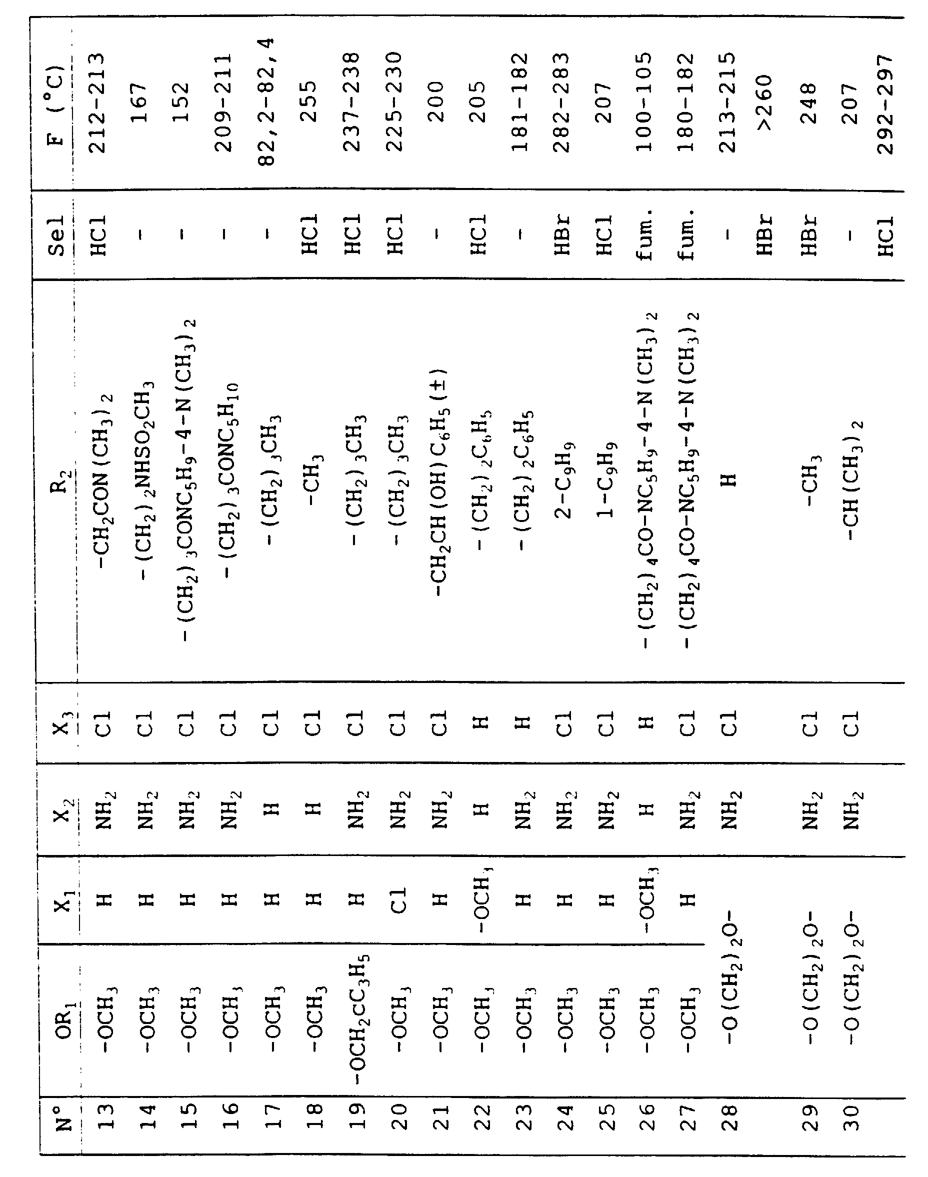

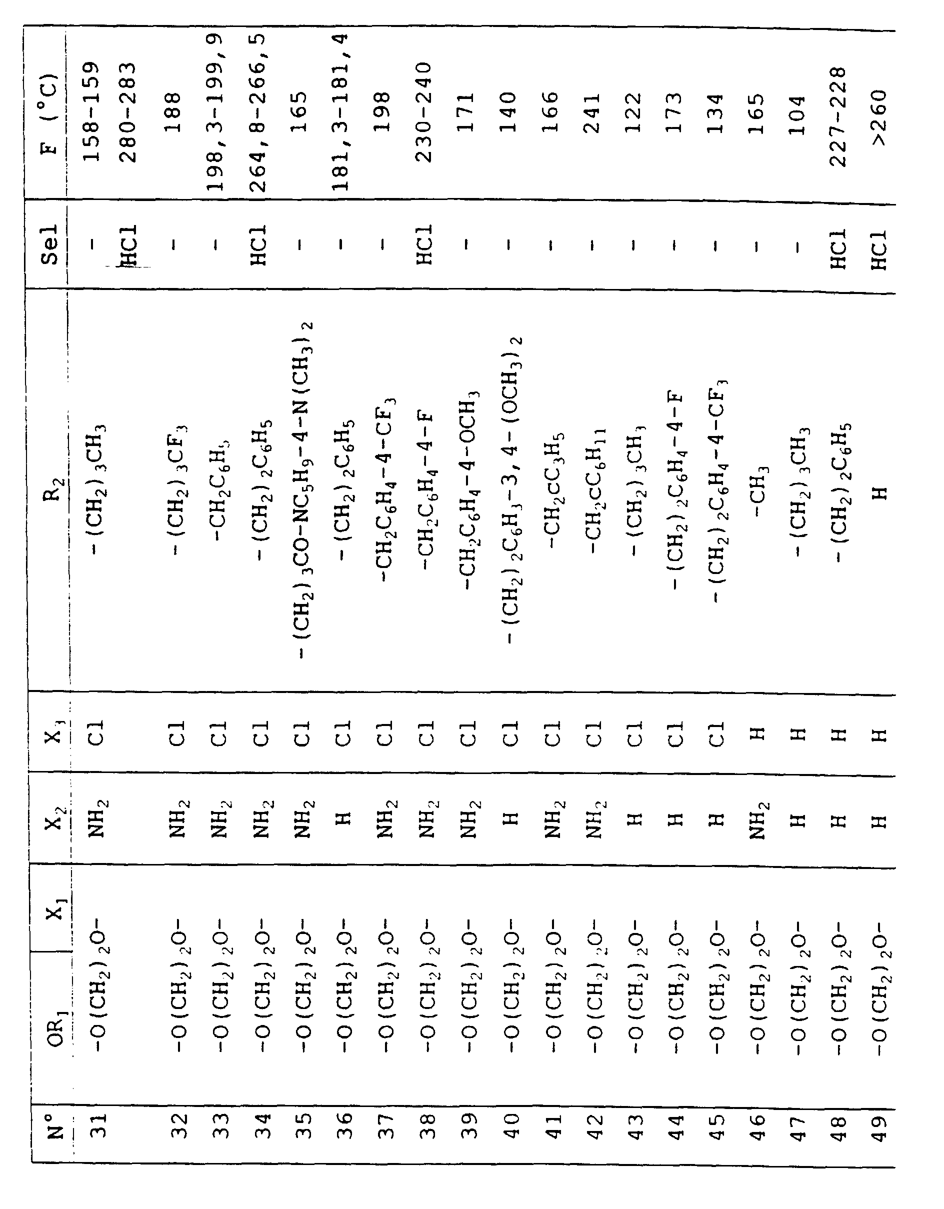

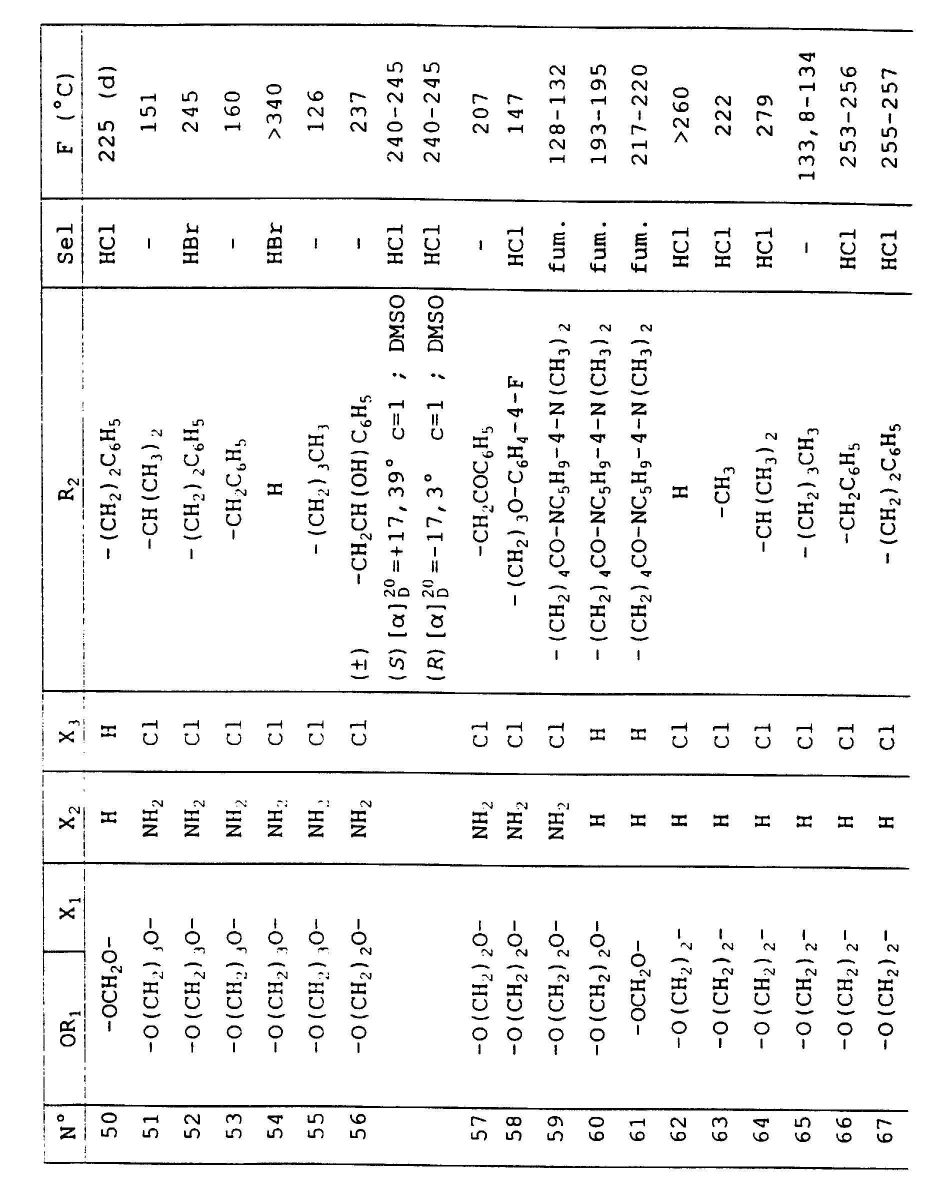

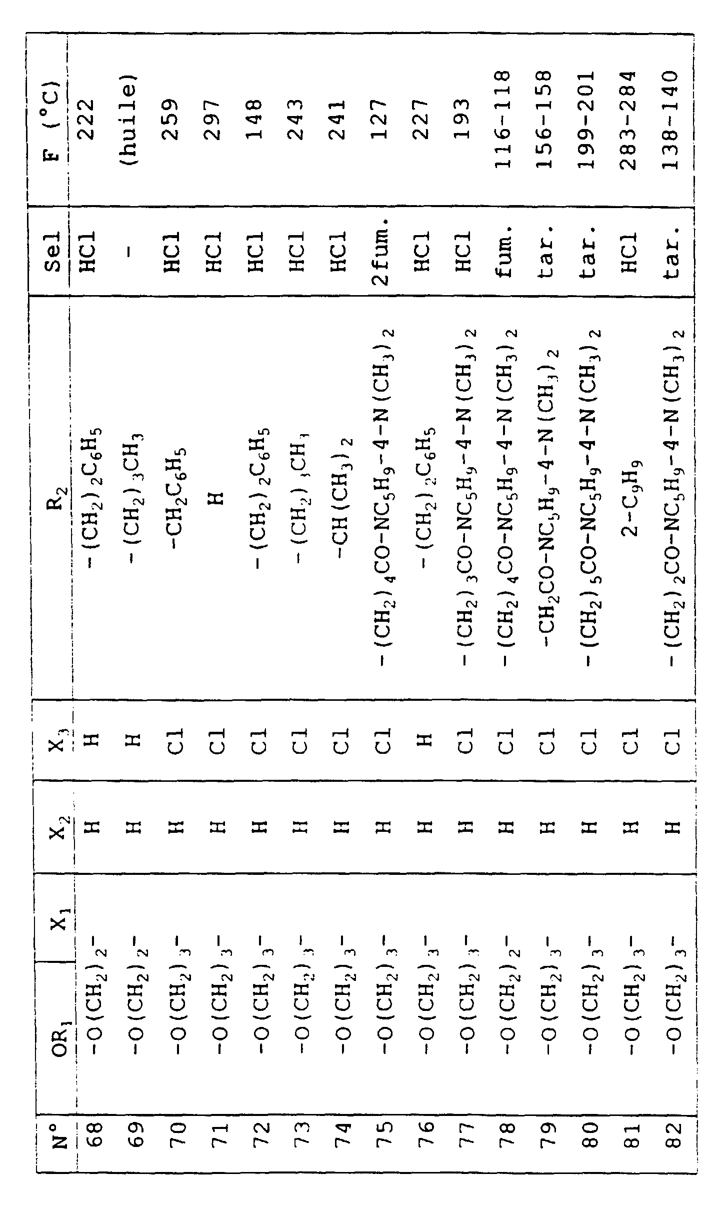

Les composés de l'invention ont fait l'objet d'essais qui ont mis en évidence leur intérêt comme substances à activités thérapeutiques.

Ainsi les composés de l'invention ont été étudiés quant à leur affinité vis-à-vis des récepteurs 5-HT₄ dans le striatum de cobaye selon la méthode décrite par Grossman et coll. dans *Br. J. Pharmacol.* (1993) **109** 618-624.
On euthanasie des cobayes (Hartley, Charles River, France) de 300 à 400 g, on prélève les cerveaux, on excise les striata et on les congèle à -80°C.
Le jour de l'expérience on décongèle le tissu à +4°C dans 33 volumes de tampon HEPES-NaOH (50 mM, pH = 7,4 à 20°C), on l'homogénéise à l'aide d'un broyeur Polytron^{™}, on centrifuge l'homogénat à 48000 g pendant 10 min, on récupère le culot, on le remet en suspension, on le centrifuge de nouveau dans les mêmes conditions et on remet le culot final en suspension dans du tampon HEPES-NaOH, à raison de 30 mg de tissu par ml. On fait incuber 100 µl de cette suspension membranaire à 0°C pendant 120 min en présence de [³H]GR113808 (ligand décrit dans l'article cité, activité spécifique 80-85 Ci/mmole) dans un volume final de 1 ml de tampon HEPES-NaOH (50 mM, pH = 7,4), en présence ou en absence de composé à tester. On arrête l'incubation par filtration sur filtre Whatman GF/B préalablement traité avec de la polyéthylèneimine à 0,1%, on rince chaque tube avec 4 ml de tampon à 0°C, on filtre de nouveau et on mesure la radioactivité retenue sur le filtre par scintigraphie liquide.
On détermine la liaison non spécifique en présence de sérotonine 30 µM. La liaison spécifique représente 90% de la radioactivité totale récupérée sur le filtre.
Pour chaque concentration de composé étudié on détermine le pourcentage d'inhibition de la liaison spécifique du [³H]GR113808 puis la CI₅₀, concentration du composé testé qui inhibe 50% de la liaison spécifique.
Les CI₅₀ des composés les plus actifs se situent entre 0,1 et 10 nM.

Les composés de l'invention ont aussi été étudiés quant à leurs effets agonistes ou antagonistes vis-à-vis des récepteurs 5-HT₄ dans l'oesophage de rat selon la méthode décrite par Baxter et coll. dans *Naunyn Schmied. Arch. Pharmacol.* (1991) **343** 439.
On utilise des rats mâles Sprague-Dawley pesant de 300 à 450 g. On prélève rapidement un fragment d'environ 1,5 cm de la partie terminale de l'oesophage, on élimine la couche musculaire, on ouvre longitudinalement la tunique muqueuse musculaire interne, on la monte dans une cuve à organe isolé contenant une solution de Krebs-Henseleit à 32°C oxygénée par un courant carbogène (95% O₂ et 5% CO₂), et on la connecte à un transducteur isométrique sous une tension basale de 0,5 g. On induit une contraction du tissu par l'addition de 0,5 µM de carbachol, on attend que la contraction se stabilise (15 min), puis on expose la préparation à la sérotonine (1 µM) afin de quantifier la relaxation maximale. On lave le tissu et, après de 20 min, on ajoute à nouveau 0,5 µM de carbachol, et on expose la préparation au composé à étudier, en concentrations cumulées croissantes de 0,1 à 1 µM. Les composés qui induisent une relaxation sont considérés comme des agonistes 5-HT₄.
Pour les composés qui n'induisent pas de relaxation, la préparation est exposée à la sérotonine en concentrations cumulées croissantes, de 0,1 nM jusqu'à une concentration induisant une relaxation maximale, et la courbe de relaxation due à la sérotonine, en présence du composé à étudier, est alors comparée à une courbe témoin établie en l'absence dudit composé. Si sa présence induit un déplacement de la courbe vers la droite, le composé étudié est alors considéré comme un antagoniste 5-HT₄.

Les résultats de ces deux essais biologiques montrent que les composés de l'invention sont de puissants ligands des récepteurs sérotoninergiques de type 5-HT₄, et qu'ils agissent sur ces récepteurs soit comme des agonistes, soit comme des antagonistes.

Enfin les composés de l'invention ont fait l'objet d'une étude *in vitro* quant à leur affinité pour les récepteurs histaminergiques H₃ du cerveau du rat, essentiellement comme décrit par Korte A. et coll., *Biochem. Phys. Res. Commun.* (1990) **168** 979-986, et West R. E. et coll., *Mol. Pharmacol.* (1990) **38** 610-613.

Des rats mâles Sprague Dawley (OFA, Iffa Credo, France), d'un poids de 250 à 300 g, sont euthanasiés et leur cerveau est prélevé. Les tissus sont homogénéisés à l'aide d'un broyeur Polytron^{™} (position 7, pendnat 20 s) dans 20 volumes de tampon Tris-HCl (50 mM, pH 7,4 à 22°C). L'homogénat est centrifugé à 1000 g pendant 10 min, puis le surnageant est soumis à une nouvelle centrifugation à 45000 g pendant 20 min à 4°C. Le culot est ensuite lavé par remise en suspension dans du tampon, homogénéisation et centrifugation. Le culot final est remis en suspension dans le tampon à raison de 100 mg de tissu initial par millilitre, puis réparti en fractions aliquotes de 11 ml, qui sont congelées à -80°C. Le jour de l'expérience, la suspension membranaire (100 µl, 300 à 400 µg de protéines) est incubée à 30°C pendant 60 min en présence de 0,5 nM de [³H]N^{α}-méthylhistamine (activité spécifique 75 à 80 Ci/mmole, New England Nuclear, Du Pont de Nemours, Boston, USA) dans un volume final de 500 µl de tampon Tris-HCl, en présence ou en absence de composé à tester. L'incubation est arrêtée par filtration sur filtres Whatman GF/B^{™} préalablement traités à la polyéthylenimine (0,4%). Chaque tube réactionnel est rincé 3 fois avec 4 ml de tampon Tris-HCl froid (0°C). Les filtres sont séchés dans une étuve à 120°C pendant 5 min. La radioactivité retenue sur les filtres est déterminée par scintigraphie liquide. La liaison non spécifique est déterminée en présence de 10 µM de thioperamide (*N*-cyclohexyl-4-(1*H*-imidazol-4-yl)pipéridine-1-carbothioamide.
Pour chaque concentration de composé étudié, le pourcentage d'inhibition de la liaison spécifique de la [³H]N^{α}-méthyl-histamine est calculé, puis la concentration CI₅₀ de composé inhibant 50% de la liaison est déterminée.

Les composés de l'invention les plus actifs dans cet essai ont une CI₅₀ de l'ordre de 5 nM.

Les résultats des divers essais biologiques effectués sur les composés de l'invention montrent qu'ils sont des ligands des récepteurs 5-HT₄ et/ou des récepteurs H₃.
Ces résultats suggèrent que les composés peuvent être utilisés pour le traitement et la prévention des désordres dans lesquels les récepteurs 5-HT₄ et/ou H₃ sont impliqués, notamment au niveau du système nerveux central, du système gastro-intestinal, du système du bas appareil urinaire ou du système cardiovasculaire.

Au niveau du système nerveux central, ces désordres et troubles comprennent notamment les troubles neurologiques et psychiatriques tels que les troubles cognitifs, les psychoses, les comportements compulsifs et obsessionnels et les états de dépression et d'anxiété. Les troubles cognitifs comprennent, par exemple, les déficits de mémoire et d'attention, les états de démence (démences séniles du type de la maladie d'Alzheimer ou démences liées à l'âge), les déficiences cérébrales vasculaires, la maladie de Parkinson. Les psychoses comprennent, par exemple, la paranoïa, la schizophrénie, la manie et l'autisme. Les comportements compulsifs et obsessionnels comprennent, par exemple, les troubles alimentaires du type de la boulimie ou de la perte d'appétit. Les états de dépression et d'anxiété comprennent, par exemple, les anxiétés de type anticipatoire (avant intervention chirurgicale, avant traitement dentaire, etc), l'anxiété causée par la dépendance ou le sevrage d'alcool ou de drogue. Enfin on peut citer encore la manie, l'épilepsie, les troubles du sommeil, les désordres affectifs saisonniers, les migraines.

Au niveau du système gastro-intestinal, ces désordres et troubles comprennent notamment les troubles directs ou indirects de la gastromotilité de l'oesophage, de l'estomac ou des intestins, les nausées, les maladies spécifiques comme la dyspepsie, l'ulcère, le reflux gastro-oesophagien, la flatulence, le syndrome du côlon irritable, les troubles de la sécrétion intestinale, les diarrhées, par exemple celles induites par le choléra ou par le syndrome carcinoïde, les désordres liés ou non à la pollution atmosphérique, tels que l'asthme, les rhinites et les difficultés respiratoires.

Au niveau du système du bas appareil urinaire, ces désordres et troubles comprennent notamment les incontinences urinaires, la dysurie, la rétention urinaire.

Au niveau du système cardiovasculaire, ces désordres et troubles comprennent notamment les pathologies liées, directement ou indirectement, aux arythmies cardiaques, à l'hypertension, à l'ischémie, à l'infarctus du myocarde, à l'angine instable, les problèmes de réocclusion après recanalisation, par exemple après thérapie fibrinolytique ou thrombolytique, angioplastie ou chirurgie coronaire. Le glaucome est également un désordre susceptible d'être traité par les composés de l'invention.

Les composés de l'invention peuvent être présentés sous toutes formes de compositions appropriées à l'administration entérale ou parentérale, telles que comprimés, dragées, gélules, capsules, suspensions ou solutions buvables ou injectables telles que sirops ou ampoules, etc, associés à des excipients convenables, et dosés pour permettre une administration journalière de 0,001 à 20 mg/kg.

## Revendications

1. Composé, éventuellement sous forme d'isomère optique pur ou de mélange de tels isomères, répondant à la formule générale (I) 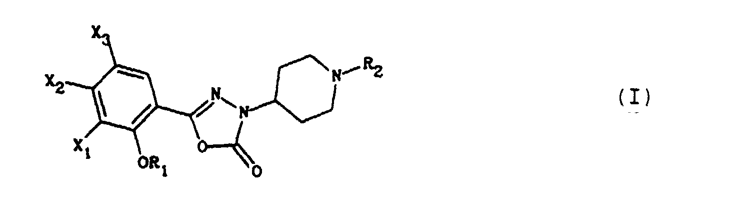 dans laquelle
R₁ représente un groupe (C₁-C₄)alkyle ou (C₃-C₇)cycloalkylméthyle,
X₁ représente un atome d'hydrogène ou d'halogène ou un groupe (C₁-C₄)alcoxy ou bien
OR₁ et X₁ représentent ensemble un groupe de formule -OCH₂O-, -O(CH₂)₂-, -O(CH₂)₃-, -O(CH₂)₂O- ou -O(CH₂)₃O-,
X₂ représente un atome d'hydrogène ou un groupe amino,
X₃ représente un atome d'hydrogène ou d'halogène, et
R₂ représente soit un atome d'hydrogène, soit un groupe (C₁-C₆)alkyle éventuellement substitué, soit un groupe phényl(C₁-C₄)alkyle éventuellement substitué sur le noyau phényle, soit un groupe phényl(C₂-C₃)alcényle, soit un groupe phénoxy(C₂-C₄)alkyle, soit un groupe cyclo(C₃-C₇)alkylméthyle, soit un groupe 2,3-dihydro-1*H*-indén-1-yle ou 2,3-dihydro-1*H*-indén-2-yle, soit un groupe de formule générale -(CH₂)ₙCO-Z dans laquelle n représente un nombre de 1 à 6 et Z représente un groupe pipéridin-1-yle ou 4-(diméthylamino)pipéridin-1-yle,
à l'état de base libre ou de sel d'addition à un acide.

2. Composé selon la revendication 1, caractérisé en ce que R₂ représente un groupe 2-éthoxy-2-oxoéthyle, un groupe 2-(diméthylamino)-2-oxoéthyle, un groupe 2-[(méthylsulfonyl)amino]éthyle, un groupe 2-oxo-2-phényléthyle, un groupe 2-hydroxy-2-phényléthyle, un groupe butyle, un groupe 4,4,4-trifluorobutyle ou un groupe 4-trifluoro-3-hydroxybutyle.

3. Composé selon la revendication 1, caractérisé en ce que R₂ représente un groupe phényl(C₁-C₃)alkyle éventuellement substitué sur le noyau phényle par un atome d'halogène, par un groupe trifluorométhyle ou par un ou deux groupes méthoxy.

4. Composé selon la revendication 1, caractérisé en ce que R₂ représente un groupe 4-oxo-4-(pipéridin-1-yl)butyle, un groupe 2-[4-(diméthylamino)pipéridin-1-yl]-2-oxoéthyle, un groupe 4-[4-(diméthylamino)pipéridin-1-yl]-4-oxobutyle, un groupe 5-[4-(diméthylamino)pipéridin-1-yl]-5-oxopentyle ou un groupe 6-[4-(diméthylamino)pipéridin-1-yl]-6-oxohexyle.

5. Procédé de préparation de composés selon la revendication 1, caractérisé en ce qu'on fait réagir un ester de formule générale (II) 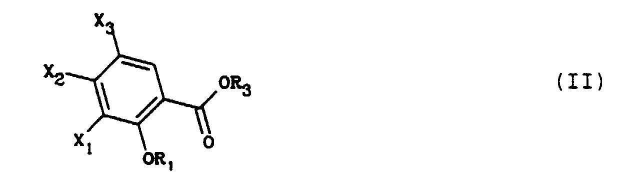 dans laquelle R₁, X₁, X₂ et X₃ sont tels que définis dans la revendication 1 et R₃ représente un groupe méthyle ou éthyle, avec l'hydrate d'hydrazine, pour obtenir un hydrazide de formule générale (III) 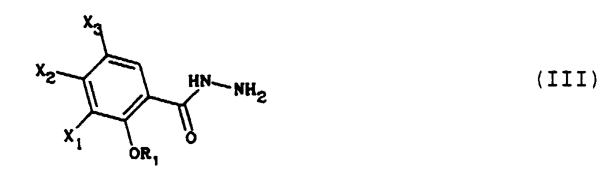 que l'on cyclise en oxadiazole de formule générale (IV) 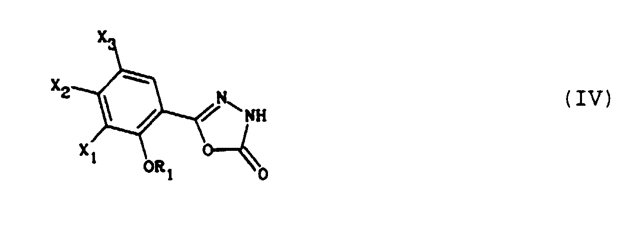 puis on fait réagir ce dernier avec un pipéridin-4-ol de formule générale (V) 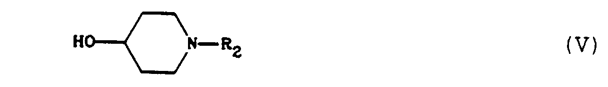 dans laquelle R₂ est tel que défini à propos de la formule générale (I), mais différent d'un atome d'hydrogène, ou bien représente un groupe protecteur (1,1-diméthyléthoxy)carbonyle, en présence de triphénylphosphine et d'azodicarboxylate d'éthyle, puis s'il y a lieu, on déprotège l'azote du cycle pipéridine et, lorsque R₂ représente un atome d'hydrogène, et si on le désire, on fait réagir le composé obtenu avec un dérivé de formule générale R₂-X, dans laquelle X représente un groupe partant ou fonctionalisable et R₂ est tel que défini à propos de la formule générale (I), mais différent d'un atome d'hydrogène, et lorsque'on désire un composé final où R₂ représente un groupe 2,3-dihydro-1*H*-indényle, on effectue un amination réductive avec un composé de formule générale (I), dans laquelle R₂ représente un atome d'hydrogène, et l'indanone correspondante.

6. Médicament caractérisé en ce qu'il est constitué d'un composé selon l'une des revendications 1 à 4.

7. Composition pharmaceutique caractérisée en ce qu'elle contient un composé selon l'une des revendications 1 à 4, associé à une excipient.

## Patentansprüche

1. Verbindung, die gegebenenfalls in Form des reinen optischen Isomeren oder einer Mischung solcher Isomeren vorliegt, der allgemeinen Formel (I) 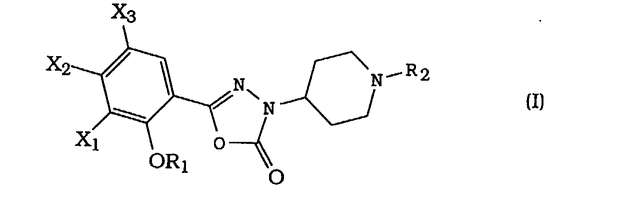 in der
R₁ eine (C₁-C₄)-Alkyl- oder (C₃-C₇)-Cycloalkylmethylgruppe,
X₁ ein Wasserstoffatom, ein Halogenatom oder eine (C₁-C₄)-Alkoxygruppe oder OR₁ und X₁ gemeinsam eine Gruppe der Formeln -OCH₂O-, -O(CH₂)₂-, -O(CH₂)₃-, -O(CH₂)₂O- oder -O(CH₂)₃O-,
X₂ ein Wasserstoffatom oder eine Aminogruppe,
X₃ ein Wasserstoffatom oder ein Halogenatom und
R₂ entweder ein Wasserstoffatom oder eine gegebenenfalls substituierte (C₁-C₆)-Alkylgruppe, oder eine gegebenenfalls am Phenylkern substituierte Phenyl-(C₁-C₄)-alkylgruppe, oder eine Phenyl-(C₂-C₃)-alkenylgruppe, oder eine Phenoxy-(C₂-C₄)-alkylgruppe, oder eine Cyclo-(C₃-C₇)-alkylmethylgruppe, oder eine 2,3-Dihydro-1*H*-inden-1-yl- oder 2,3-Dihydro, 1*H*-inden-2-yl-gruppe, oder eine Gruppe der allgemeinen Formel -(CH₂)ₙCO-Z, in der n eine Zahl mit einem Wert von 1 bis 6 und Z eine Piperidin-1-yl- oder 4-(Dimethylamino)-piperidin-1-yl-gruppe darstellen, bedeuten,
in Form der freien Base oder eines Säureadditionssalzes.

2. Verbindung nach Anspruch 1, dadurch gekennzeichnet, daß R₂ eine 2-Ethoxy-2-oxoethylgruppe, eine 2-(Dimethylamino)-2-oxoethylgruppe, eine 2-[(Methylsulfonyl)-amino]-ethylgruppe, eine 2-Oxo-2-phenylethylgruppe, eine 2-Hydroxy-2-phenylethylgruppe, eine Butylgruppe, eine 4,4,4-Trifluorbutylgruppe oder eine 4-Trifluor-3-hydroxybutylgruppe bedeutet.

3. Verbindung nach Anspruch 1, dadurch gekennzeichnet, daß R₂ eine Phenyl-(C₁-C₃)-alkylgruppe, die gegebenenfalls am Phenylkern durch ein Halogenatom, eine Trifluormethylgruppe oder eine oder zwei Methoxygruppen substituiert ist, bedeutet.

4. Verbindung nach Anspruch 1, dadurch gekennzeichnet, daß R₂ eine 4-Oxo-4-(piperidin-1-yl)-butylgruppe, eine 2-[4-(Dimethylamino)-piperidinl-1-yl]-2-oxo-ethylgruppe, eine 4-[4-(Dimethylamino)-piperidin-1-yl]-4-oxobutylgruppe, eine 5-[4-(Dimethylamino)-piperidin-1-yl]-5-oxopentylgruppe oder eine 6-[4-(Dimethylamino)-piperidin-1-yl]-6-oxohexylgruppe bedeutet.

5. Verfahren zur Herstellung der Verbindungen nach Anspruch 1, dadurch gekennzeichnet, daß man einen Ester der allgemeinen Formel (II) 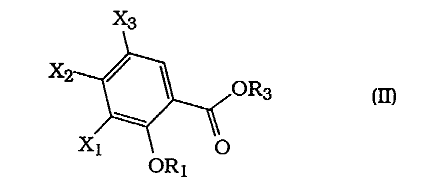 in der R₁, X₁, X₂ und X₃ die in Anspruch 1 angegebenen Bedeutungen besitzen und R₃ eine Methyl- oder Ethylgruppe darstellt, mit Hydrazinhydrat umsetzt zur Bildung eines Hydrazids der allgemeinen Formel (III) 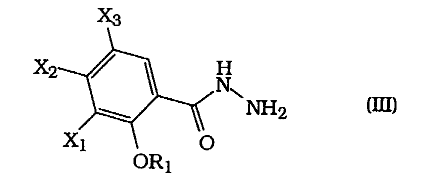 welches man zu einem Oxadiazol der allgemeinen Formel (IV) cyclisiert 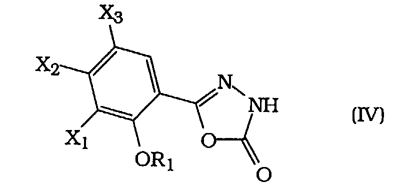 welch letztere Verbindung man mit einem Piperidin-4-ol der allgemeinen Formel (V) 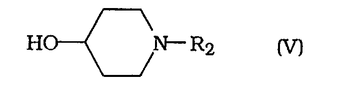 in der R₂ die bezüglich der allgemeinen Formel (I) angegebenen Bedeutungen besitzt mit Ausnahme des Wasserstoffatoms, oder eine (1,1-Dimethylethoxy)-carbonylschutzgruppe darstellt, in Gegenwart von Triphenylphosphin und Azodicarbonsäureethylester umsetzt und erforderlichenfalls die Schutzgruppe am Stickstoffatom des Piperidinrings abspaltet und, wenn R₂ ein Wasserstoffatom darstellt, man gewünschtenfalls die erhaltene Verbindung mit einem Derivat der allgemeinen Formel R₂-X, in der X eine austretende Gruppe oder eine funktionalisierbare Gruppe bedeutet und R₂ die bezüglich der allgemeinen Formel (I) angegebenen Bedeutungen besitzt, jedoch von einem Wasserstoff verschieden ist, umsetzt und, wenn eine Endverbindung hergestellt werden soll, in der R₂ eine 2,3-Dihydro-1*H*-indenylgruppe darstellt, man eine reduktive Aminierung mit einer Verbindung der allgemeinen Formel (I), in der R₂ ein Wasserstoffatom darstellt, und dem entsprechenden Indanon durchführt.

6. Arzneimittel, dadurch gekennzeichnet, daß es aus einer Verbindung nach einem der Ansprüche 1 bis 4 gebildet ist.

7. Pharmazeutische Zubereitung, dadurch gekennzeichnet, daß sie eine Verbindung nach einem der Ansprüche 1 bis 4 zusammen mit einem Trägermaterial enthält.

## Claims

1. Compound, optionally in the form of a pure optical isomer or of a mixture of such isomers, corresponding to the general formula (I) 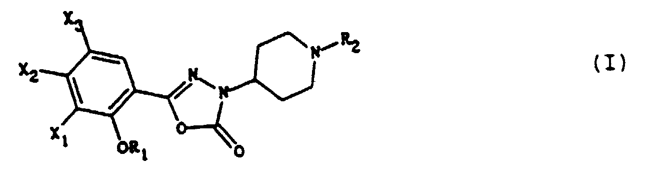 in which
R₁ represents a (C₁-C₄)alkyl or (C₃-C₇)cycloalkylmethyl group,
X₁ represents a hydrogen or halogen atom or a (C₁-C₄)alkoxy group or else
OR₁ and X₁ together represent a group of formula -OCH₂O-, -O(CH₂)₂-, -O(CH₂)₃-, -O(CH₂)₂O- or -O(CH₂)₃O-,
X₂ represents a hydrogen atom or an amino group,
X₃ represents a hydrogen or halogen atom, and
R₂ represents either a hydrogen atom or an optionally substituted (C₁-C₆)alkyl group or a phenyl(C₁-C₄)alkyl group which is optionally substituted on the phenyl ring or a phenyl (C₂-C₃)alkenyl group or a phenoxy(C₂-C₄) alkyl group or a cyclo(C₃-C₇)alkylmethyl group or a 2,3-dihydro-1*H*-inden-1-yl or 2,3-dihydro-1*H*-inden-2-yl group or a group of general formula -(CH₂)ₙCO-Z in which n represents a number from 1 to 6 and Z represents a piperidin-1-yl or 4-(dimethylamino)piperidin-1-yl group,
in the form of the free base or of an addition salt with an acid.

2. Compound according to Claim 1, characterized in that R₂ represents a 2-ethoxy-2-oxoethyl group, a 2-(dimethylamino)-2-oxoethyl group, a 2-[(methylsulphonyl)amino]ethyl group, a 2-oxo-2-phenylethyl group, a 2-hydroxy-2-phenylethyl group, a butyl group, a 4,4,4-trifluorobutyl group or a 4-trifluoro-3-hydroxybutyl group.

3. Compound according to Claim 1, characterized in that R₂ represents a phenyl(C₁-C₃)alkyl group optionally substituted on the phenyl ring by a halogen atom, by a trifluoromethyl group or by one or two methoxy groups.

4. Compound according to Claim 1, characterized in that R₂ represents a 4-oxo-4-(piperidin-1-yl)butyl group, a 2-[4-(dimethylamino)piperidin-1-yl]-2-oxoethyl group, a 4-[4-(dimethylamino)piperidin-1-yl]-4-oxobutyl group, a 5-[4-(dimethylamino)piperidin-1-yl]-5-oxopentyl group or a 6-[4-(dimethylamino)piperidin-1-yl]-6-oxohexyl group.

5. Process for the preparation of compounds according to Claim 1, characterized in that an ester of general formula (II) 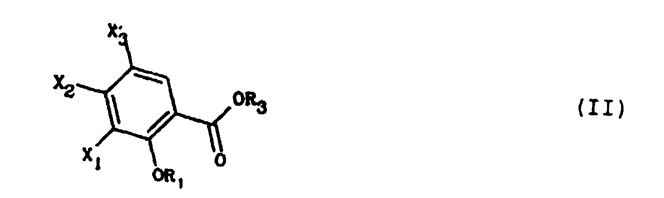 in which R₁, X₁, X₂ and X₃ are as defined in Claim 1 and R₃ represents a methyl or ethyl group, is reacted with hydrazine hydrate, in order to obtain a hydrazide of general formula (III) 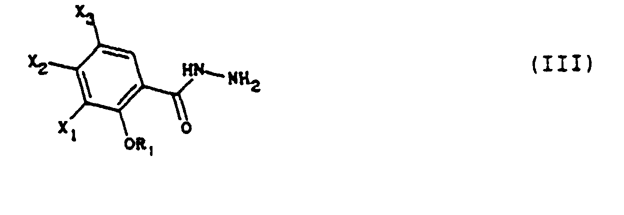 which is cyclized to the oxadiazole of general formula (IV) 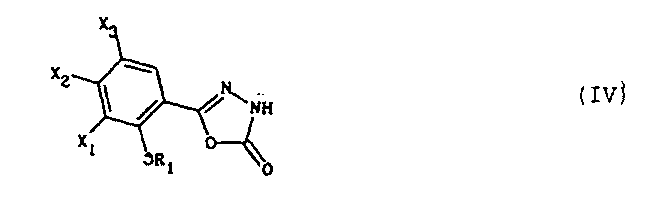 the latter is then reacted with a piperidin-4-ol of general formula (V) 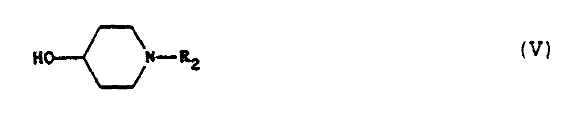 in which R₂ is as defined with respect to the general formula (I) but is other than a hydrogen atom or else represents a (1,1-dimethylethoxy)carbonyl protective group, in the presence of triphenylphosphine and ethyl azodicarboxylate, then, if it takes place, the nitrogen of the piperidine ring is deprotected and, when R₂ represents a hydrogen atom and if it is desired, the compound obtained is reacted with a derivative of general formula R₂-X, in which X represents a leaving or functionalizable group and R₂ is as defined with respect to the general formula (I) but is other than a hydrogen atom, and, when a final compound is desired where R₂ represents a 2,3-dihydro-1*H*-indenyl group, a reductive amination is carried out with a compound of general formula (I), in which R₂ represents a hydrogen atom, and the corresponding indanone.

6. Medicament, characterized in that it is composed of a compound according to one of Claims 1 to 4.

7. Pharmaceutical composition, characterized in that it contains a compound according to one of Claims 1 to 4, in combination with an excipient.
